# EUROPÄISCHE PATENTSCHRIFT

(11) **EP 2 047 248 B1**
(45) Veröffentlichungstag und Bekanntmachung des Hinweises auf die Patenterteilung: **09.02.2011**
(21) Anmeldenummer: 07785575.7
(22) Anmeldetag: 28.06.2007
(51) Int. Cl.: G01N 33/12, G01N 9/02

(54) **VERFAHREN UND VORRICHTUNG ZUR BESTIMMUNG DES FETTGEHALTES EINER GESAMTHEIT VON FLEISCHSTÜCKEN**
METHOD AND DEVICE FOR DETERMINING THE FAT CONTENT OF PIECES OF MEAT IN THEIR ENTIRETY
PROCÉDÉ ET DISPOSITIF POUR DÉTERMINER LA TENEUR EN GRAISSE DE MORCEAUX DE VIANDE DANS LEUR ENSEMBLE

(30) Priorität: 13.07.2006 DE 102006032423
(43) Veröffentlichungstag der Anmeldung: 15.04.2009
(73) Patentinhaber: Delipetkos, Elias, 65549 Limburg (DE)
(72) Erfinder: Delipetkos, Elias, 65549 Limburg (DE)
(74) Vertreter: Patentanwälte Lambsdorff & Lange
(86) Internationale Anmeldenummer: PCT/DE2007/001142
(87) Internationale Veröffentlichungsnummer: WO 2008/006336

(56) Entgegenhaltungen:
- DE-A1- 2 220 110
- DE-A1- 4 200 770
- DE-A1- 10 064 707
- US-A- 4 449 406
- US-A1- 2002 014 116

## Beschreibung

Die vorliegende Erfindung betrifft ein Verfahren und eine Vorrichtung zur Bestimmung des durchschnittlichen Fettgehaltes von in einem Behälter aufbewahrten Fleischstücken.

Die Erfindung bezieht sich generell auf das Gebiet der Kontrolle und Prüfung von Lebensmittelprodukten und speziell auf das Gebiet der Kontrolle von Fleischprodukten. Letztere müssen heutzutage je nach ihrer Bestimmung festgelegte Bereiche hinsichtlich ihres Fettgehalts einhalten. Aus diesem Grund ist eine möglichst genaue Bestimmung des Fettgehalts bei Fleischprodukten und gegebenenfalls eine entsprechende Einstellung des Fettgehalts als Reaktion auf einen gemessenen Fettgehalt im Produktionsprozess von erheblicher Bedeutung. In früheren Produktions- und Verarbeitungsanlagen konnte eine Analyse des Fettgehaltes von Fleisch nur diskontinuierlich durch Entnahme einer Fleischprobe aus einem Mischer oder von einem Förderband erfolgen, die dann in einem Labor analysiert wurde. Seit einiger Zeit kann jedoch die Fettanalyse bereits kontinuierlich erfolgen, indem beispielsweise der Fettgehalt eines Fleischproduktes auf einem Förderband mittels einer Detektoreinrichtung bestimmt wird.

Die Druckschrift DE 100 64 707 A1 beschreibt eine Vorrichtung zur kontinuierlichen Bestimmung des Fettgehalts von Fleisch, welche aus einem Förderband, einer Wiegezelle, einer Röntgenquelle und einem Röntgendetektor besteht. Das Fleisch wird mit dem Förderband an der Röntgenquelle vorbeigeführt und dabei von den Röntgenstrahlen durchstrahlt. Der Röntgendetektor misst die Absorption der Röntgenstrahlen durch das Fleisch und kann anhand dieser Informationen den Fettgehalt bestimmen. Gleichzeitig während der Messung wird das Gewicht des Fleisches, das sich zum Zeitpunkt der Messung auf dem Förderband befindet, gemessen und dadurch die Menge an Fleisch, die den gemessenen Fettgehalt aufweist, bestimmt. Diese Daten werden an einen Zentralrechner übermittelt, sodass entweder der genaue Fettgehalt eines bestimmten Produktes bestimmt werden kann oder diese Daten zur Berechnung des Fettgehaltes beispielsweise in einem Mischer herangezogen werden können.

Die Druckschrift DE 600 10 499 T2 beschreibt ein Verfahren und eine Vorrichtung zur Bestimmung der Eigenschaften von Lebensmitteln oder Tierfutter, wobei ebenfalls im Wesentlichen der Fettgehalt von Fleisch bestimmt werden soll. Bei diesem Verfahren wird das Medium durch Röntgenstrahlen mit mindestens zwei Energiepegeln abgetastet und die durch das Medium hindurchgetretenen Röntgenstrahlen ortsaufgelöst für eine Vielzahl von Bereichen des Mediums erfasst. Für jeden dieser Bereiche wird das Röntgenabsorptionsvermögen bei den beiden verschiedenen Energiepegeln berechnet. Anhand eines bereits bestehenden Kalibrierungsmodell, welches auf einem vorbestimmten Zusammenhang zwischen dem Fettgehalt des Mediums und dem Röntgenabsorptionsvermögen beruht, kann dann aus den berechneten Werten für das Absorptionsvermögen der Fettgehalt des Mediums für die einzelnen durchstrahlten Bereiche bestimmt werden.

Eine Bestimmung des Fettgehalts von gehacktem Fleisch anhand seines Volumengewichtes wird in DE 2220110 beschrieben, wobei das Volumen mithilfe von Druckluft gemessen wird.

Die im Stand der Technik beschriebenen Verfahren und Vorrichtungen ermöglichen zwar im Prinzip eine kontinuierliche, in einen Produktionsprozess integrierbare Analyse des Fettgehalts von Fleischprodukten. Die bekannten Konzepte weisen jedoch im wesentlichen noch die Nachteile auf, dass sie entweder nicht in der Lage sind, die Analyse des Fettgehalts mit der nötigen Präzision ermitteln zu können, oder dass sie zwar einen bestimmten Präzisionsgrad erreichen, jedoch von der technischen Implementierung zu aufwändig sind. Insbesondere ist noch kein zufriedenstellendes Konzept bekannt, nach welchem ein mit einer Anzahl von Fleischprodukten gefüllter Behälter hinsichtlich des Fettgehalts der Fleischprodukte in einem kontinuierlichen Prozess analysiert und bewertet werden kann.

Es ist somit Aufgabe der vorliegenden Erfindung, ein Verfahren und eine Vorrichtung zur Bestimmung des durchschnittlichen Fettgehaltes von in einem Behälter aufbewahrten Fleischstücken anzugeben, durch welche der durchschnittliche Fettgehalt schnell und präzise bei vertretbarem technischen Implementierungsaufwand bestimmt werden kann.

Diese Aufgabe wird durch die Merkmale der unabhängigen Patentansprüche gelöst. Vorteilhafte Weiterbildungen und Ausgestaltungen sind Gegenstand von Unteransprüchen.

In der fleischverarbeitenden Industrie tritt häufig das Bedürfnis auf, den durchschnittlichen Fettgehalt einer Gesamtheit von Fleischstücken zu bestimmen und auf der Basis des Ergebnisses einer entsprechenden Analyse eine Entscheidung darüber zu fällen, ob die Gesamtheit der Fleischstücke aufgrund eines zu geringen durchschnittlichen Fettgehalts ausgeschlossen wird oder dem weiteren Prozess, insbesondere dem Vertrieb oder Verkauf, zugeführt wird. Es geht dabei also in erster Linie darum, den durchschnittlichen Fettgehalt der Gesamtheit der Fleischstücke zu bestimmen. Erst in zweiter Linie kann dann noch versucht werden, zu analysieren, welche Teile oder Bereiche der Gesamtheit der Fleischstücke einen zu geringen Fettgehalt aufweisen. Die Gesamtheit der Fleischstücke ist dabei in einem Norm-Behälter untergebracht, der unter der Bezeichnung Satte gebräuchlich ist. Eine sehr gebräuchliche Satte mit einer bestimmten Normgröße trägt beispielsweise die Bezeichnung E2-Satte.

Das erfindungsgemäße Verfahren zur Bestimmung des durchschnittlichen Fettgehaltes von in einem Behälter aufbewahrten Fleischstücken weist die Schritte auf
a) Bestimmen des Gesamtgewichtes der Fleischstücke,
b) Bestimmen des Verlaufs der Oberfläche der in dem Behälter aufbewahrten Fleischstücke und Bestimmen eines von der Gesamtheit der Fleischstücke eingenommenen Volumens aus dem bestimmten Verlauf der Oberfläche, und
c) Bestimmen des Fettgehaltes aus den bestimmten Werten für das Gesamtgewicht und das Volumen.

Eine bevorzugte Weiterbildung des erfindungsgemäßen Verfahrens besteht in einem zusätzlichen Schritt d. des Bestimmens des durchschnittlichen Röntgenabsorptionsvermögens der in dem Behälter aufbewahrten Fleischstücke.

Die Erfindung geht somit von der grundlegenden und allgemein bekannten Tatsache aus, dass Fett leichter ist als Fleisch und somit eine Bestimmung des Fettgehalts im Prinzip bereits durch eine Gewichtsmessung möglich sein könnte. Dies würde jedoch voraussetzen, dass in den Behältern bzw. Satten stets exakt gleiche Gesamtvolumina an Fleisch enthalten sind, was natürlich nicht der Fall ist, da sowohl die Anzahl der Fleischstücke als auch deren Grössen variieren können. Erfindungsgemäß ist somit vorgesehen, zumindest näherungsweise das Gesamtvolumen der in dem Behälter aufbewahrten Fleischstücke zu bestimmen. Die Fleischstücke sind in der Regel in dem Behälter bis zu einer bestimmten Füllhöhe übereinander gestapelt. In der Nähe der Füllhöhe wird durch die zu oberst liegenden Fleischstücke eine Oberfläche gebildet, deren Verlauf bzw. Topografie bestimmt werden kann. Aus diesem bestimmten Verlauf der Oberfläche kann dann ein Näherungswert für das von der Gesamtheit der Fleischstücke angenommene Volumen bestimmt werden. Dieser Näherungswert ergibt sich aus der Grösse des zwischen dem gemessenen Oberflächenverlauf und den Begrenzungswänden des Behälters liegenden Volumens. Aus den solchermaßen bestimmten Größen für das Gewicht und das Volumen kann dann auf den durchschnittlichen Fettgehalt der in dem Behälter aufbewahrten Fleischstücke geschlossen werden. Dies kann beispielsweise so erfolgen, indem die beiden Grössen Gewicht und Volumen geeignet miteinander verknüpft werden (Multiplikation oder Division) und die verknüpfte Grösse auf eine vorab bestimmte Kalibrationskurve zwischen dem Fettgehalt und der verknüpften Grösse angewandt wird. Diese Kalibrationskurve kann aus anderen präzisen Fettanalyseverfahren wie beispielsweise chemischen Fettanalyseverfahren vorab gewonnen erzeugt worden sein. Die verknüpfte Grösse kann beispielweise der Quotient Volumen/Gewicht sein, da diese Grösse zumindest näherungsweise proportional zu dem Fettgehalt sein sollte.

Es versteht sich, dass die vorgenannten Kalibrationen und Berechnungen sich anstatt auf den Fettgehalt ebenso auf die zu dem Fettgehalt reziproke Grösse Magergehalt beziehen können.

Es ist durchaus möglich, dass das bis hierhin beschriebene Verfahren es bereits ermöglicht, den durchschnittlichen Fettgehalt der in dem Behälter enthaltenen Fleischstücke mit hinreichender Genauigkeit innerhalb vorgegebener Toleranzspannen zu bestimmen. In folgender Hinsicht können jedoch die Bestimmungen der Messgrößen Gewicht und Volumen fehlerhaft sein, sodass auch der daraus bestimmte Fettgehalt mit einem Fehler behaftet ist. Zum einen wird wie bereits erwähnt nur ein Näherungswert für das Gesamtvolumen der Fleischstücke bestimmt, indem die von diesen oder einem Teil von diesen gebildete Oberfläche in ihrem Verlauf bestimmt wird. Es kann nämlich sein, dass - bedingt durch die Stapelung der Fleischstücke in dem Behälter - ein verfälschtes Volumen bestimmt wird. Beispielsweise können die Fleischstücke so gestapelt sein, dass zwischen ihnen relativ viele Leerräume gebildet werden, sodass eine Füllhöhe und eine entsprechende Lage und Verlauf der Oberfläche gemessen werden, die höher sind als bei einer anderen Art der Stapelung der Fleischstücke. Somit wird ein Volumenwert bestimmt, welcher höher ist als ein tatsächlicher Wert für das Gesamtvolumen der Fleischstücke. Zum anderen können auch bei der Gesamtgewichtsbestimmung der Fleischstükke Fehler auftreten. Diese Gewichtsbestimmung wird im Regelfall so durchgeführt werden, dass das Gewicht des Behälters mit den darin aufbewahrten Fleischstücken gemessen wird und von dem gemessenen Gewichtswert das bekannte Gewicht des Behälters abgezogen wird. Da aber hinsichtlich des bekannten Behältergewichts beispielsweise stest ein einheitlicher Normwert verwendet wird und nicht jeder Behälter einzeln in seinem Gewicht gemessen wird, können Fehler entstehen, da die Behälter in der Praxis unterschiedliche Gewichtswerte aufweisen können.

Aus den vorgenannten Gründen besteht eine bevorzugte Weiterbildung des erfindungsgemäßen Verfahrens darin, dass zusätzlich noch das durchschnittliche Röntgenabsorptionsvermögen der in dem Behälter aufbewahrten Gesamtheit der Fleischstücke bestimmt wird. In diesem Fall ist darauf zu achten, dass der Behälter aus einem Material mit einer geringen Absorption für Röntgenstrahlung im Vergleich mit den zu untersuchenden Fleischstücken hergestellt ist. Da auch das Förderband, auf welchem der Behälter transportiert wird, von der Röntgenstrahlung durchstrahlt werden muss, sollte auch dieses aus einem Material mit geringer Röntgenabsorption bestehen. Die Röntgenstrahlung wird beispielsweise oberhalb des Behälters bzw. des Förderbandes von einer geeigneten Röntgenstrahlquelle erzeugt und kegelförmig nach unten in Richtung auf den Behälter und die darin aufbewahrten Fleischstücke abgestrahlt. Unterhalb des Behälters bzw. des Förderbandes befindet sich ein Röntgendetektor für die Aufnahme der durch die Gesamtheit der Fleischstücke durchgetretenen Röntgenstrahlung. Der Röntgendetektor kann in bekannter Weise als Flächendetektor ausgebildet sein, sodass eine ortsaufgelöste Detektion der durch die Fleischstücke durchgetretenen Röntgenstrahlung möglich ist. Entsprechend der Ortsauflösung des Röntgendetektors kann dann auch der Behälter mit den darin aufbewahrten Fleischstücken in eine matrixförmige Anordnung von säulenförmigen Bereichen unterteilt werden, für die dann jeweils mittels des Röntgendetektors die Intensität der durch sie durchgetretenen Röntgenstrahlung detektiert wird. Zusätzlich ist aus dem Verfahrensschritt b., in welchem der Verlauf der Oberfläche bestimmt wurde, die Länge und somit das Volumen von jedem der säulenförmigen Bereiche bekannt, sodass aus der gemessenen Intensität der durchgetretenen Röntgenstrahlung ein Absorptionskoeffizient für jeden der säulenförmigen Bereiche bestimmt werden kann. Dieses ist dann ein über das gesamte Volumen des säulenförmigen Bereichs gemittelter Absorptionskoeffizient. Aus dem gemittelten Absorptionskoeffizienten kann dann der Fettgehalt in dem säulenförmigen Bereich bestimmt werden. In an sich bekannter Weise kann dies beispielsweise wieder anhand einer zuvor aufgenommenen Kalibrierungsfunktion geschehen. Diese kann aufgenommen werden, indem durch Fettanalyseverfahren wie chemische Fettanalyseverfahren der Fettgehalt verschiedener Fleischproben über einen grösseren Bereich bestimmt wird und dazu deren Röntgen-Absorptionskoeffizienten gemessen, werden.

Mit dem Röntgendetektor ist es somit möglich, innerhalb bestimmter Grenzen den Fettgehalt in der Gesamtheit der in dem Behälter aufbewahrten Fleischstücke ortsaufgelöst zu bestimmen. Abgesehen davon kann natürlich die gesamte durch die Fleischstücke durchgetretene Röntgenstrahlung durch den Röntgendetektor in ihrer Intensität gemessen werden und unter der Kenntnis des von der Röntgenstrahlung durchsetzten Volumens ein mittlerer Röntgenabsorptions-Koeffizient bestimmt werden. Dieser über die Gesamtheit des Volumens der Fleischstücke gemittelte Röntgen-Absorptionskoeffizient kann dann wieder, wie oben bereits für die säulenförmigen Bereiche angedeutet, mit Hilfe vorher aufgenommener Kalibrierungsfunktionen in einen Fettgehalt umgerechnet werden.

Die zusätzlich durchgeführte Röntgenabsorptionsmessung kann somit sehr vorteilhaft dafür eingesetzt werden, um ein aus den vorherigen Messungen des Gewichts und des Volumens erzieltes Zwischenergebnis hinsichtlich des Fettgehalts der Gesamtheit der Fleischstücke zu verifizieren oder möglicherweise auch zu falsifizieren. Gegebenenfalls sind die Messergebnisse aus allen drei genannten Messungen hinsichtlich des Gewichts, des Volumens und der Röntgenabsorption in geeigneter Weise miteinander zu kombinieren, um mit Hilfe einer geeigneten vorher aufgenommenen Kalibrierungsfunktion den durchschnittlichen Fettgehalt der Fleischstücke zu bestimmen.

Es kann vorab ein bestimmter Schwellwert für einen durchschnittlichen Fettgehalt festgelegt werden. Insofern der mit dem erfindungsgemäßen Verfahren bestimmte durchschnittliche Fettgehalt der in der Satte enthaltenen Fleischportionen überschritten wird, wird die Satte nach Durchlaufen der Analysestationen ausgeschleust. Wird der Schwellwert dagegen eingehalten oder unterschritten, so wird die Satte weiterverarbeitet bzw. ihrer Bestimmung, insbesondere für den Vertrieb oder Verkauf, präpariert.

Die Röntgenabsorptionsmessung kann darüberhinaus dafür verwendet werden, in den Fleischprodukten enthaltene Fremdkörper zu detektieren. Für die Entscheidung, ob die Satte ausgeschleust oder verwendet wird, kommt somit zusätzlich zu dem Fettgehalte noch ein weiteres Kriterium hinsichtlich der Detektion etwaiger Fremdkörper hinzu.

Der Verfahrensschritt b. des Bestimmens des Verlaufs der Oberfläche kann darin bestehen, dass die Oberfläche berührungslos abgetastet wird. Dabei kann ein Strahlungsbündel auf die Oberfläche gerichtet werden und die von der Oberfläche zurückgeworfene Strahlung ausgewertet werden. Dies kann bevorzugtermaßen durch ein elektromagnetisches Strahlungsbündel wie ein Laser-Strahlungsbündel und einen entsprechenden Detektor für die zurückgestreute Laserstrahlung erfolgen. Alternativ dazu kann auch als Strahlungsbündel ein Ultraschall-Strahlungsbündel verwendet werden, wobei dann geeignete Ultraschall-Detektoren für die zurückgeworfene oder reflektierte Ultraschall-Strahlung anzuordnen sind.

Mit dem Aussenden und Empfangen des Strahlungsbündels wird die Entfernung zwischen der Abtastungsvorrichtung und der Oberfläche gemessen. Da mit dem Strahlungsbündel die Oberfläche abgerastert wird, kann somit die Position und der Verlauf der Oberfläche in allen drei Raumrichtungen bestimmt werden, wobei die Ortsauflösung durch die Rastergeschwindigkeit und die Geschwindigkeit bestimmt wird, mit der durch die Detektionseinrichtung die rückgestreute Strahlung aufgenommen wird.

Die Erfindung bezieht sich ebenso auf eine Vorrichtung zur Bestimmung des durchschnittlichen Fettgehaltes von in einem Behälter aufbewahrten Fleischstücken, welche aufweist
- eine Fördervorrichtung zum Fördern des Behälters,
- eine Wiegevorrichtung zum Bestimmen des Gewichtes der Fleischstücke,
- eine Verlaufsbestimmungs-Vorrichtung zur Bestimmung des Verlaufs der Oberfläche der in dem Behälter aufbewahrten Fleischstücke, und
- eine Recheneinrichtung zum Bestimmen des Volumens der Fleischstücke auf der Basis des Verlaufs der Oberfläche und zum Bestimmen des Fettgehaltes aus den bestimmten Größen, Gewicht und Volumen.

Die Erfindung wird im Folgenden anhand eines Ausführungsbeispiels unter Bezugnahme auf die Zeichnungen näher erläutert. Es zeigen:
- Fig. 1: eine Draufsicht auf eine schematische Darstellung eines Ausführungsbeispiels für eine erfindungsgemä- ße Vorrichtung;
- Fig. 2: eine Seitenansicht einer schematischen Darstellung für die Durchführung des Abtastverfahrens zur Be- stimmung des Verlaufs der Oberfläche; und
- Fig. 3: eine perspektivische Ansicht einer schematischen Darstellung für die Durchführung des Röntgenanaly- se-Verfahrens.

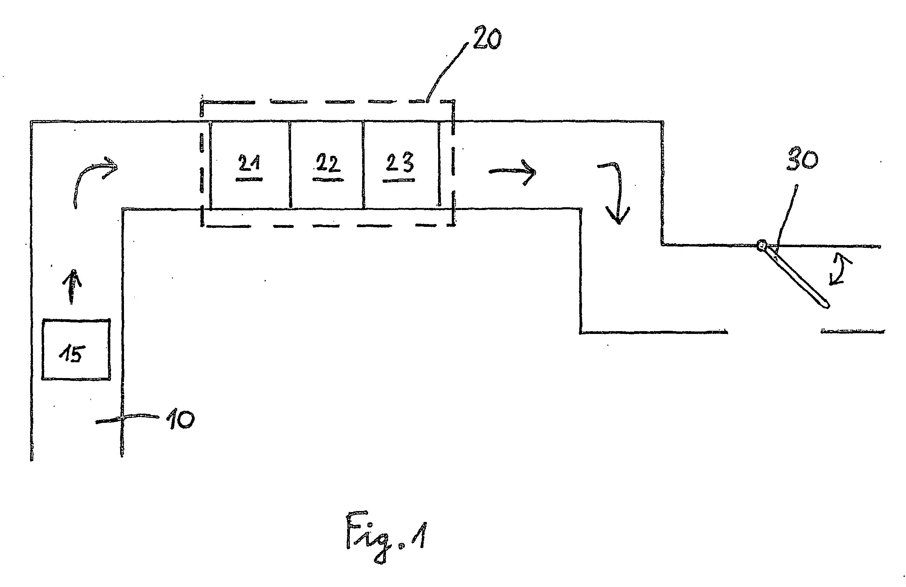

In der Fig. 1 ist eine Draufsicht auf eine schematische Darstellung eines Ausführungsbeispiels für eine erfindungsgemäße Vorrichtung gezeigt. Auf einem Förderband 10 wird ein Behälter 15, insbesondere eine sogenannte Satte, mit einer Anzahl von darin aufbewahrten, gestapelten Fleischstücken befördert. Der Behälter 15 wird durch das Förderband 10 zu einer Analysevorrichtung 20 befördert. Die Analysevorrichtung 20 weist eine Wiegezelle 21, eine Laserscan-Einrichtung 22 und eine Röntgenanalyseeinrichtung 23 auf. Die genannten Einrichtungen sind mit einer Datenverarbeitungseinrichtung (nicht dargestellt) gekoppelt. Nach Durchlaufen der Analysevorrichtung 20 wird der Behälter 15 durch das Förderband 10 zu einer Ausschleusereinrichtung 30 befördert. Die Ausschleusereinrichtung 30 wird durch die Datenverarbeitungseinrichtung angesteuert. Sofern durch die von der Analysevorrichtung 20 an die Datenverarbeitungseinrichtung gelieferten Messergebnisse und die von der Datenverarbeitungseinrichtung durchgeführten Berechnungen festgestellt wurde, dass der durchschnittliche Fettgehalt der Gesamtheit der in dem Behälter 15 enthaltenen Fleischstücke oberhalb eines vorgegebenen Schwellwertes liegt und/oder durch die Röntgenanalyseeinrichtung 23 Fremdkörper detektiert wurden, wird die Ausschleusereinrichtung 30 aktiviert, um den Behälter 15 auszuschleusen.

In der Wiegezelle 21 wird das Gewicht des Behälters 15 mit den darin gestapelten Fleischstücken gemessen und der gemessene Wert an die Recheneinrichtung weitergeleitet. In der Recheneinrichtung wird aus diesem Wert das Gesamtgewicht der Fleischstücke durch Subtrahieren des Behältergewichts berechnet.

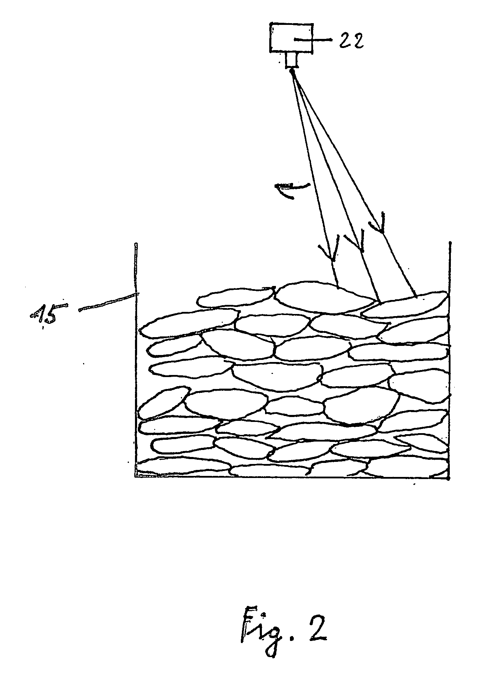

In der Fig. 2 ist eine Seitenansicht des Behälters 15 und der in dem Behälter gestapelten Fleischstücke und der Laserscan-Einrichtung 22 dargestellt. Durch die oberste Lage der Fleischstücke wird eine Oberfläche der Gesamtheit der Fleischstücke gebildet, deren Verlauf oder Topografie mit der Laserscan-Einrichtung 22 bestimmt werden soll. Zu diesem Zweck wird die Oberfläche durch den Laserstrahl der Laserscan-Einrichtung 22 abgetastet und die von jedem abgetasteten Punkt der Oberfläche zurückgeworfene Strahlung durch einen Detektor (nicht dargestellt) der Laserscan-Einrichtung 22 analysiert und solchermassen die Lage und der Verlauf der Oberfläche innerhalb des Behälters 15 bestimmt. Von jedem abgetasteten Punkt werden aus der von dem Punkt rückgestreuten Strahlung die räumlichen Koordinaten durch Entfernungsmessung von der Abtasteinrichtung 22 ermittelt. Aus dem solchermaßen ermittelten Verlauf der Oberfläche kann dann ein Näherungswert für das Gesamtvolumen der Gesamtheit der Fleischstücke gewonnen werden. Dieser Näherungswert ist durch dasjenige Volumen gegeben, welches durch den abgetasteten Verlauf der Oberfläche und die sich unterhalb davon anschließenden Begrenzungswände des Behälters 15 bestimmt.

Die Laserscan-Einrichtung bzw. der in ihr enthaltene Detektor 22 liefert beispielsweise die von ihm aufgenommenen Rohdaten hinsichtlich der Entfernungsmessungen der abgetasteten Punkte der Oberfläche an die Recheneinrichtung und in der Recheneinrichtung werden dann die Ortskoordinaten der abgetasteten Punkte berechnet und aus der sich daraus ergebenden Position und Verlauf der Oberfläche wird dann der Näherungswert des Volumens unter Hinzunahme der Begrenzungswände des Behälters 15 berechnet.

Gegebenenfalls schliesst sich daran bereits die Ermittlung des Fettgehalts aus dem Gesamtgewicht und dem Volumen und einer in der Recheneinrichtung enthaltenen Kalibrationsfunktion an.

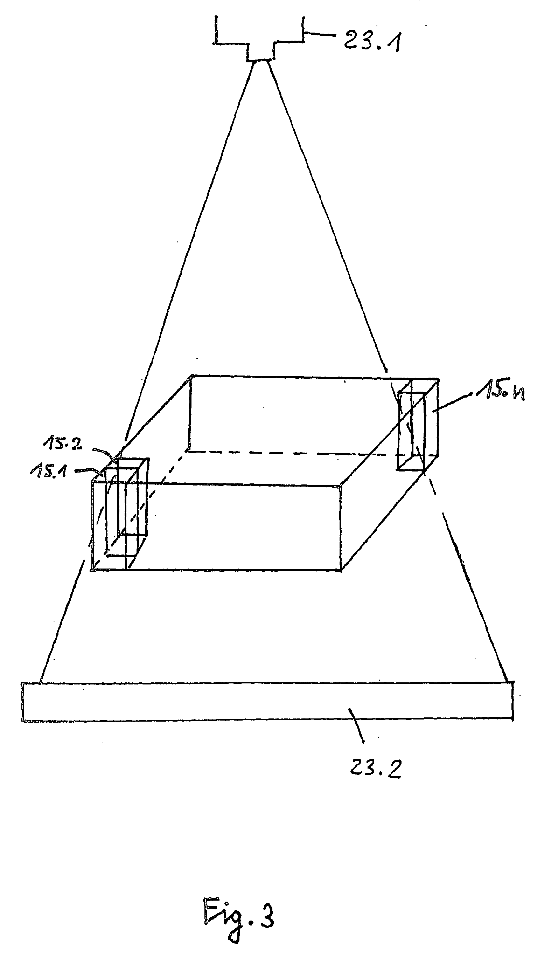

Es kann jedoch noch eine Röntgenabsorptionsmessung durchgeführt werden. In der Fig. 3 ist der Behälter 15 und eine Röntgenanalyse-Einrichtung 23 perspektivisch dargestellt. Die Röntgenanalyse-Einrichtung 23 weist eine Röntgenstrahlquelle 23.1 und einen Röntgendetektor 23.2 auf. Durch die Röntgenstrahlquelle 23.1 wird ein kegelförmiges Röntgenstrahlungsbündel in Richtung auf den Behälter 15 abgestrahlt und der Röntgendetektor 23.2, der einen Flächendetektor enthält, detektiert ortsaufgelöst die durch den Behälter 15 durchgetretene Röntgenstrahlung. Entsprechend der Ortsauflösung des Röntgendetektors 23.2 kann der Behälter 15 in eine matrixförmige Anordnung säulenförmiger Bereich 15.1 ... 15.n unterteilt werden, für die jeweils die durch sie durchgetretene Röntgenstrahlung und daraus ein mittlerer Röntgen-Absorptionskoeffizient bestimmt werden kann.

Es kann dann ein über alle Bereiche 15.1 ... 15.n gemittelter Röntgen-Absorptionskoeffizient ermittelt werden und mittels einer Kalibrationsfunktion kann aus diesem gemittelten Röntgen-Absorptionskoeffizienten ein Fettgehalt ermittelt werden.

## Patentansprüche

1. Verfahren zur Bestimmung des durchschnittlichen Fettgehaltes von in einem Behälter aufbewahrten Fleischstücken, mit den Schritten
a. Bestimmen des Gesamtgewichtes der Fleischstücke,
b. Bestimmen des Verlaufs der Oberfläche der in dem Behälter aufbewahrten Fleischstücke und Bestimmen eines von der Gesamtheit der Fleischstücke eingenommenen Volumens aus dem bestimmten Verlauf der Oberfläche, und
c. Bestimmen des Fettgehaltes aus den bestimmten Größen Gewicht und Volumen.

2. Verfahren nach Anspruch 1, mit dem zusätzlichen Schritt d. Bestimmen des durchschnittlichen Röntgenabsorptionsvermögens der Fleischstücke.

3. Verfahren nach Anspruch 1 oder 2, bei welchem im Schritt b. die Oberfläche berührungslos abgetastet wird.

4. Verfahren nach Anspruch 3, bei welchem ein Strahlungsbündel auf die Oberfläche gerichtet und die von der Oberfläche zurückgeworfene Strahlung ausgewertet wird.

5. Verfahren nach Anspruch 4, bei welchem das Strahlungsbündel ein elektromagnetisches Strahlungsbündel, insbesondere ein Laser-Strahlungsbündel, oder ein Ultraschall-Strahlungsbündel ist.

6. Verfahren nach einem der vorhergehenden Ansprüche, bei welchem im Schritt a. das Gewicht des die Fleischstücke aufbewahrenden Behälters bestimmt wird und von dem bestimmten Wert das bekannte Gewicht des Behälters abgezogen wird.

7. Vorrichtung zur Bestimmung des durchschnittlichen Fettgehaltes von in einem Behälter (15) aufbewahrten Fleischstükken, mit
- einer Fördervorrichtung (10) zum Fördern des Behälters (15),
- einer Wiegevorrichtung (21) zum Bestimmen des Gewichtes der Fleischstücke,
- einer Verlaufsbestimmungs-Vorrichtung (22) zur Bestimmung des Verlaufs der Oberfläche der in dem Behälter (15) aufbewahrten Fleischstücke,
- einer Recheneinrichtung eingerichtet zum Bestimmen des Volumens der Fleischstücke auf der Basis des Verlaufs der Oberfläche und zum Bestimmen des Fettgehaltes aus den bestimmten Größen Gewicht und Volumen.

8. Vorrichtung nach Anspruch 7, mit
- einer Röntgenanalyse-Vorrichtung (23) zur Bestimmung des durchschnittlichen Röntgenabsorptionsvermögen der Fleischstücke.

9. Vorrichtung nach Anspruch 7 oder 8, bei welcher die Verlaufsbestimmungs-Vorrichtung (22) eine Abtastungsvorrichtung zur berührungslosen Abtastung der Oberfläche aufweist.

10. Vorrichtung nach Anspruch 9, bei welcher die Abtastungsvorrichtung eine Strahlungseinrichtung zur Erzeugung und Aussendung eines Strahlungsbündels in Richtung auf die Oberfläche und eine Detektionseinrichtung zur Detektion der von der Oberfläche zurückgeworfenen Strahlung aufweist.

11. Vorrichtung nach Anspruch 10, bei welcher die Strahlungseinrichtung eine Strahlungseinrichtung zur Erzeugung eines elektromagnetischen Strahlungsbündels, insbesondere eine Laser-Einrichtung, oder eine Ultraschall-Strahlungseinrichtung ist.

## Claims

1. A method for determining the average fat content of pieces of meat held in a container, comprising the steps:
a. determining the total weight of the pieces of meat
b. mapping the surface of the pieces of meat held in the container and determining a volume taken up by the pieces of meat in their entirety from the mapped surface, and
c. determining the fat content from the determined weight and volume variables.

2. The method as set forth in claim 1 comprising the additional step
d. determining the average x-ray absorption capacity of the pieces of meat.

3. The method as set forth in claim 1 or 2 wherein in step b. the surface is proximity scanned.

4. The method as set forth in claim 3 wherein a radiation beam is directed at the surface and the radiation backscattered from the surface is analyzed.

5. The method as set forth in claim 4 wherein said radiation beam is an electromagnetic radiation beam, particularly a laser radiation beam or ultrasound radiation beam.

6. The method as set forth in any of the preceding claims wherein in step a. the weight of the container holding the pieces of meat is included, from which the known weight of the container is deducted.

7. A device for determining the average fat content of pieces of meat held in a container (15), comprising
- a conveyor device (10) for conveying the container (15),
- a weighing device (21) for determining the weight of the pieces of meat,
- a mapping device (22) for mapping the surface of the pieces of meat held in the container (15),
- a computer device programmed to determine the volume of the pieces of meat on the basis of the mapped surface and to determine the fat content from the determined weight and volume variables.

8. The device as set forth in claim 7 comprising
- an x-ray analyzing device (23) for determining the average x- ray absorption capacity of the pieces of meat.

9. The device as set forth in claim 7 or 8 wherein said mapping device (22) comprises a surface proximity scanning device.

10. The device as set forth in claim 9 wherein said scanning device comprises a radiation device for generating and emitting a radiation beam in the direction of the surface and a detection device for detecting the radiation backscattered from the surface.

11. The device as set forth in claim 10 wherein said radiation device is a radiation device for generating an electromagnetic radiation beam, particularly a laser radiation device or ultrasound radiation device.

## Revendications

1. Procédé pour la détermination de la teneur moyenne en graisse des morceaux de viande conservés dans un récipient, comportant les étapes consistant à :
a. déterminer le poids total des morceaux de viande,
b. déterminer la continuité de la surface des morceaux de viande conservés dans le récipient et déterminer un volume adopté par la totalité des morceaux de viande à partir de la continuité déterminée de la surface, et
c. déterminer la teneur en graisse à partir des grandeurs déterminées à savoir le poids et le volume.

2. Procédé selon la revendication 1, comprenant l'étape supplémentaire consistant à : d. déterminer le pouvoir d'absorption moyen des rayons X des morceaux de viande.

3. Procédé selon l'une ou l'autre des revendications 1 et 2, dans lequel la surface est balayée sans contact au cours de l'étape « b ».

4. Procédé selon la revendication 3, dans lequel un faisceau de rayons est dirigé vers la surface et dans lequel le rayonnement réfléchi en retour par la surface est évalué.

5. Procédé selon la revendication 4, dans lequel le faisceau de rayons est un faisceau de rayons électromagnétiques, en particulier un faisceau de rayons laser ou un faisceau de rayons aux ultrasons.

6. Procédé selon l'une des revendications précédentes, dans lequel le poids du récipient qui conserve les morceaux de viande est déterminé au cours de l'étape « a. » et dans lequel le poids connu du récipient est soustrait de la valeur déterminée.

7. Dispositif pour déterminer la teneur moyenne en graisse des morceaux de viande conservés dans un récipient (15), comprenant :
- un dispositif de convoyage (10) afin de convoyer le récipient (15),
- un dispositif de pesage (21) afin de déterminer le poids des morceaux de viande,
- un dispositif de détermination de la continuité (22) afin de déterminer la continuité de la surface des morceaux de viande conservés dans le récipient (15),
- un dispositif de calcul mis au point afin de déterminer le volume des morceaux de viande sur la base de la continuité de la surface et afin de déterminer la teneur en graisse à partir des grandeurs déterminées à savoir le poids et le volume.

8. Dispositif selon la revendication 7, comprenant :
- un dispositif d'analyse des rayons X (23) pour la détermination du pouvoir d'absorption moyen des rayons X des morceaux de viande.

9. Dispositif selon l'une ou l'autre des revendications 7 et 8, dans lequel le dispositif de détermination de la continuité (22) présente un dispositif de balayage pour le balayage sans contact de la surface.

10. Dispositif selon la revendication 9, dans lequel le dispositif de balayage présente un dispositif de rayonnement afin de générer et d'émettre un faisceau de rayons en direction de la surface et un dispositif de détection pour la détection du rayonnement réfléchi par la surface.

11. Dispositif selon la revendication 10, dans lequel le dispositif de rayonnement est un dispositif de rayonnement afin de générer un faisceau de rayons électromagnétiques, en particulier un dispositif laser ou un dispositif de rayonnement aux ultrasons.
